# EUROPEAN PATENT SPECIFICATION

(11) **EP 1 214 926 B1**
(45) Date of publication and mention of the grant of the patent: **14.03.2007**
(21) Application number: 01124783.0
(22) Date of filing: 17.10.2001
(51) Int. Cl.: A61K 8/68, A61K 8/35, A61K 8/67, A61Q 19/08

(54) **Topical composition**
Zusammensetzung zur topischen Anwendung
Composition à usage topique

(30) Priority: 12.12.2000 JP 2000376831
(43) Date of publication of application: 19.06.2002
(73) Proprietor: Kabushiki Kaisha Kishohin Kagaku Kaiho Kenkyujo, Takata-gun, Shizuoka-ken (JP)
(72) Inventor: Tashiro, Hiroko, Izunagaoka-cho, Takata-gun, Shizuoka-ken (JP); Ishida, Kazuto, Izunagaoka-cho, Takata-gun, Shizuoka-ken (JP)
(74) Representative: Betten & Resch

(56) References cited:
- EP-A- 0 276 151
- EP-A- 0 646 370
- EP-A- 0 835 655
- EP-A- 1 031 348
- WO-A-97/47289
- WO-A1-94/00127
- WO-A1-98/30215
- WO-A1-98/52536
- WO-A2-00/32155
- GB-A- 2 089 785
- JP-A- 8 157 333
- JP-A- 9 077 665
- JP-A- 2001 199 843
- US-A- 5 236 950
- FEY H.; OTTE I.: 'Wörterbuch der Kosmetik', 1997, WISSENSCHAFTLICHE VERLAGSGESELLSCHAFT MBH, STUTTGART 4th edition 1997; ISBN 3-8047-1464-1 * page 50 *
- PETERSEN R.D.: 'Ceramides, Key Components for Skin Protection' COSMETICS & TOILETRIES vol. 107, February 1992, pages 45 - 49, XP000749868

## Description

### BACKGROUND OF THE INVENTION

### 1. Field of the Invention

The present invention relates to a external dermal agent, particularly a external dermal agent effectively preventing and improving dry skin and rough skin, and wrinkle formation and the like due to loss of skin tautness and elasticity.

### 2. Description of the Related Art

Skin problems such as dry skin, rough skin, and wrinkles due to loss of skin tautness and elasticity are induced not only by aging and exposure to sunlight, but also by nutrient deficiencies of the skin due to poor circulation, irregular lifestyle, ineffective skin care, environmental factors such as cold weather and dry air, and so on.

Several drug components have been developed for the purpose of preventing and improving problems such as dry skin, rough skin, and wrinkles and the like.

Recently, the technique of improving and preventing wrinkles with retinol, vitamin A acids and derivatives thereof has been used for preventing and improving these problems such as dry skin, rough skin, and wrinkles. However, the effects of improving and preventing problems such as wrinkles are not yet sufficient.

Therefore, a need exists for the development of more effective external dermal agents.

In consideration of the above background and in order to solve the above problems, other external agents including various components extracted from natural products have been developed, and methods of smoothing wrinkles physically and temporarily by use of membrane forming agents have been utilized. However, these agents and methods are not yet sufficient.

### SUMMARY OF THE INVENTION

Accordingly, it is an object of the present invention to provide an external dermal agent capable of preventing and/or improving skin problems such as dry skin, rough skin, and wrinkles and the like due to loss of skin tautness and elasticity.

After intensive research by the inventors of the present invention to obtain a substance effective in improving skin problems such as dry skin, rough skin, and wrinkles due to loss of skin tautness and elasticity, using safe substances in order to accomplish the above object, it has been found that a composition including vitamin A, a hinokitiol, and an epidermal lipid solves the problems and achieves the object of the invention.

### BRIEF DESCRIPTION OF THE DRAWING

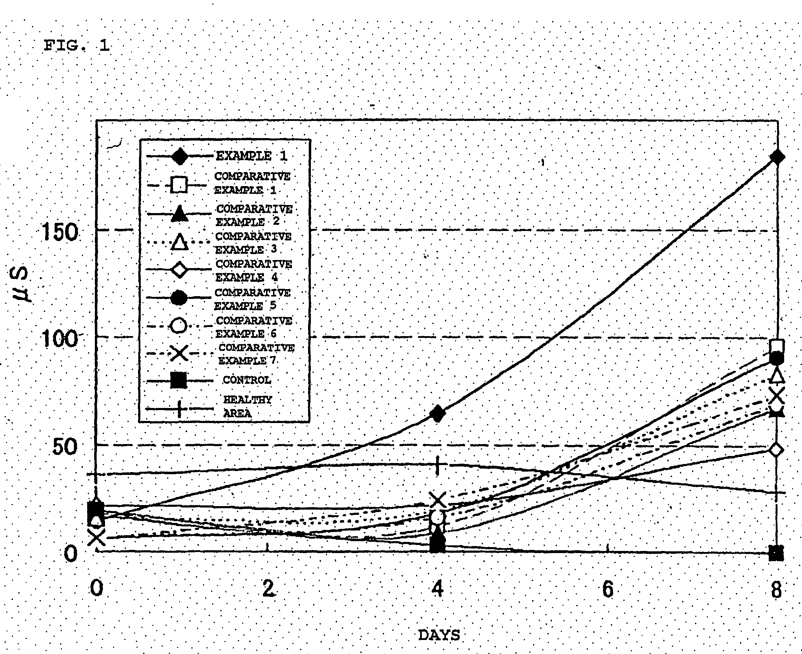

Fig. 1 is a graph showing the variations with time of the corneum moisture content in the rough skin samples shown in Table 3.

### DESCRIPTION OF THE PREFERRED EMBODIMENTS

The present invention provides a external dermal agent comprising: (A) 0,001-5,0wt% of a vitamin A compound and/or a derivative thereof; (B) 0,0001 -2,0 wt% of a hinokitiol and/or a derivative thereof; and 0,01-20,0 wt% of (C) an epidermal lipid.

The present invention is described in detail as follows.

The vitamin A compounds used in the present invention include compounds having vitamin A activity and are generally utilized as active elements for prevention and therapy of skin keratosis and the like, and furthermore for the prevention and recovery of skin aging, particularly wrinkles and the like. Among the compounds, retinol, retinal, retinoic acid, isomers thereof, and derivatives thereof (for example, retinol palmitate and retinol acetate) are particularly preferable.

The contents of the vitamin A compound included in the external dermal agent of the present invention are 0.001-5.0 wt %, and more preferably 0.01-1.0 wt %.

Hinokitiol used in the present invention is tropolone compound such as beta-thujaplicin, occurring in nature and extracted from essential oils of trees such as Aomori Hiba (*Thujopsis dotabrata Sied. et Zucc. var. hondai Makino)* and Taiwan Hinoki (*Chamaecyparis Taiwanensis*, *Masamune et. Suzuki*). The hinokitiols, whether natural compounds or synthetic compounds, are utilized widely and generally in pharmaceutical and cosmetic fields as fungicides, cell activation agents and the like.

Hinokitiol and derivatives are especially preferable among the compounds.

The contents of the hinokitiol included in the external dermal agent of the present invention are 0.0001-2.0 wt %, and more preferably 0.0005-0.5 wt %.

Lipids are utilized widely and generally in pharmaceutical and cosmetic fields as base and emollient agents, including Sterols and/or derivatives thereof, sphingolipids and/or derivatives thereof, phospholipids and/or derivatives thereof, and glycerides and/or derivatives thereof.

Also, the sterols include cholesterol, dehydrocholesterol, ergosterol, sitosterol, campesterol, stigmasterol, brassicasterol, fucosterol, and the like, and esters thereof as a derivative.

Also, the sphingolipids include sphingosine, sphingomyelin, sphingoglycolipid, cerebroside, phytosphingosine, ceramide 1, ceramide 2, ceramide 3, ceramide 4, ceramide 5, ceramide 6, and the like, and derivatives thereof.

Also, the phospholipids include phosphatidyl choline, lysophosphatidyl choline, phosphatidylethanolamine, phosphatidyl inositol, and the like, and derivatives thereof.

Also, the glycerides include fats and oils occurring naturally in plants and animals and derivatives thereof, including monoglycerides, diglycerides, triglycerides, and the like. The epidermal lipids used in the present invention are common lipids existing in the skin.

The contents of the epidermal lipids and/or the similar components included in the external dermal agent of the present invention are 0.01-20.0 wt %, and more preferably 0.1-10.0 wt %.

In addition to the above mentioned essential components, the external dermal agents of the present invention may further include, if necessary, various conventional components generally used in pharmaceutical and cosmetic fields, quasi drugs, and the like such as aqueous components, humectants, thickeners, ultraviolet light absorbers, ultraviolet light scattering agents, aseptic agents, antioxidants, flavors, coloring materials, medical agents, herbal medicines, and the like, providing the effects of the present invention are not impaired.

Also, the external dermal agent of the present invention may be prepared in many forms, for example, liquid formulations such as lotions and the like, emulsions such as milky lotions, creams, and the like, ointments, compositions including powders, water/oil two-phase type agents, water/oil/powder three-phase type agents and the like.

### EXAMPLE

The invention is further illustrated by reference to the following examples. These examples are not meant to limit the scope of the invention. In these examples, each agent was prepared by adding an oil phase heated to 80°C to an aqueous phase heated to the same temperature, stirring to emulsify, and cooling the mixture to room temperature.

The compositions described below were prepared and the effects on rough skin improvements were evaluated. It is known that dry skin causes rough skin, which progresses and leads to wrinkles and deep lines.

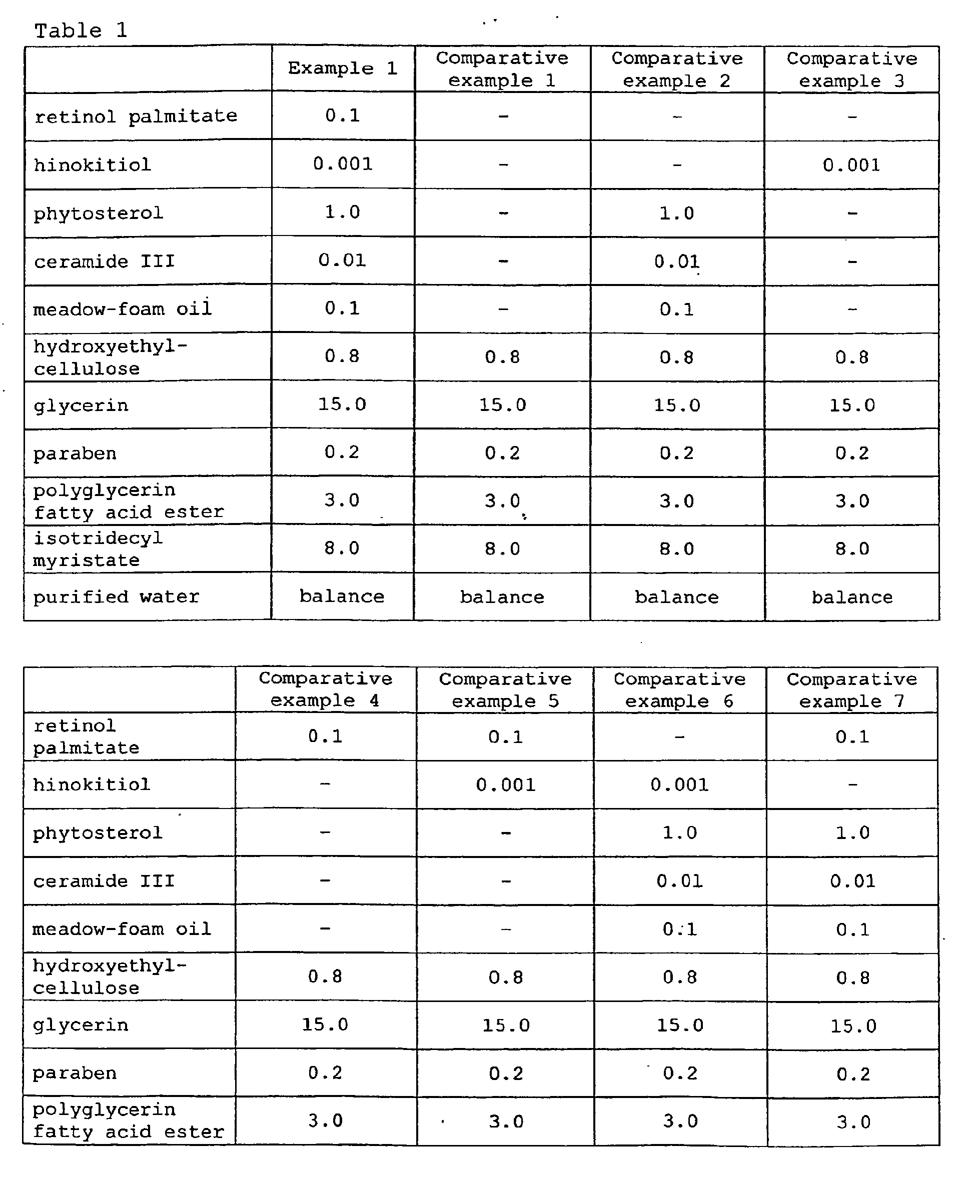

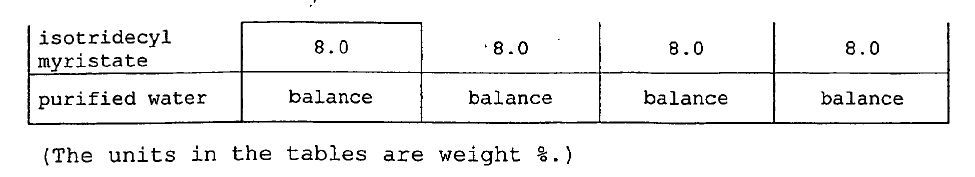

### <Method of rough skin improvement test>

The samples of rough skin area are prepared by applying films which has absorbed 10% solution of sodium lauryl sulfate to 3 cm square areas of human forearm skin, taking off the films six hours later and washing the areas by water. Then samples of 0.05 g of the Example 1 and the Comparative examples 1-7 were applied to the rough skin testing areas three times a day. After four days and after eight days, the rough skin testing areas were examined with the naked eye and then the corneum moisture contents were measured to evaluate the improvements with a skin surface corneum moisture measuring device, SKICON-200 (IBS Corporation). The results are shown in Tables 2 and 3

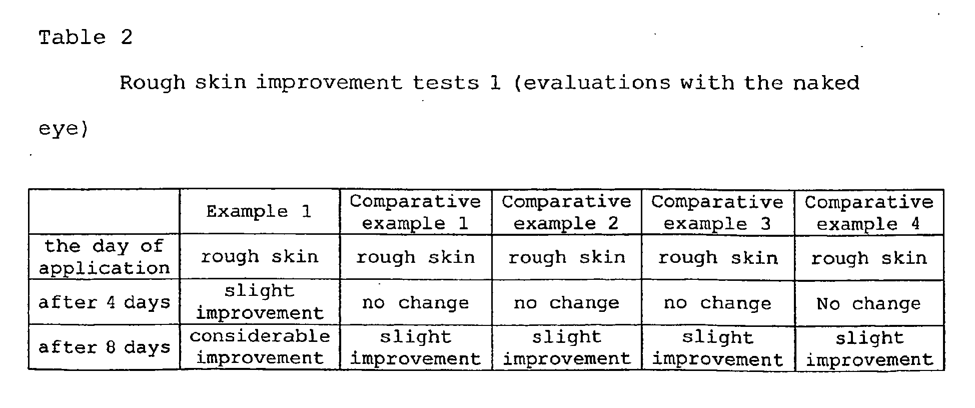

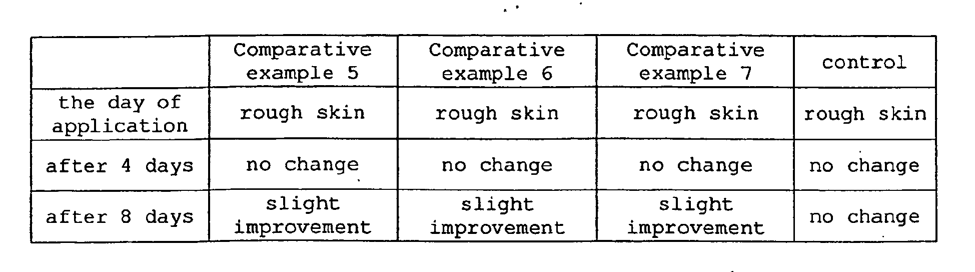

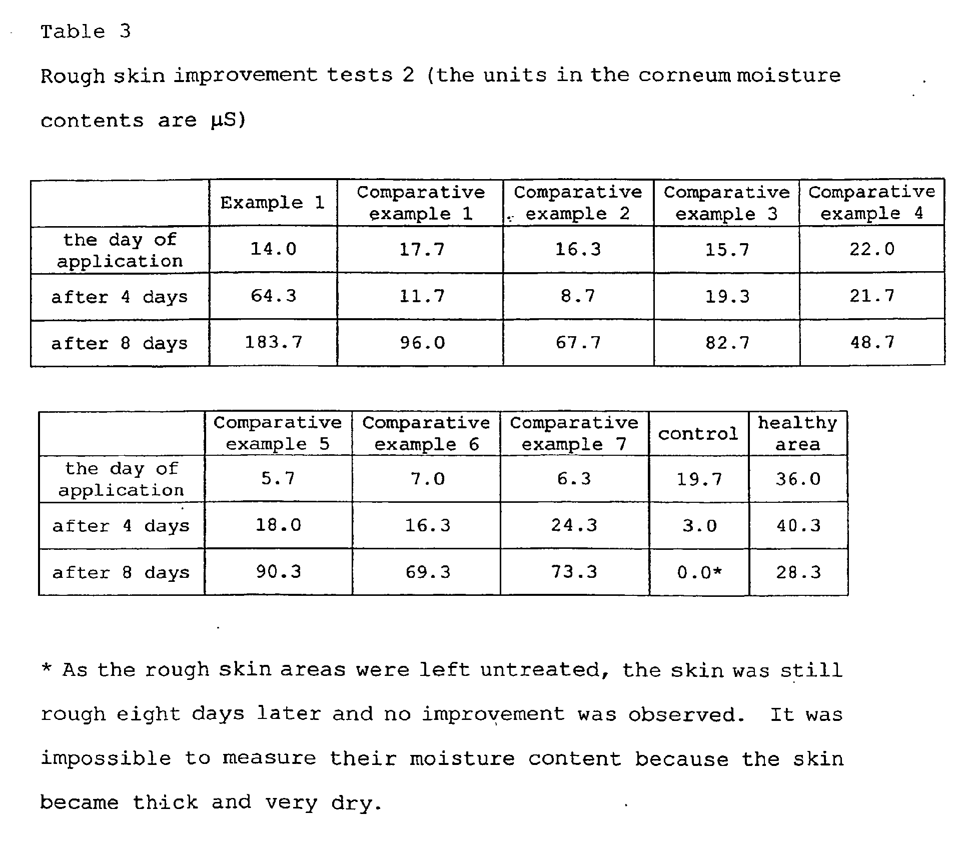

The results of Tables 2 and 3 revealed that Example 1 according to the present invention was much more effective at rough skin improvement compared with Comparative examples 1-7.

In another experiment, the compositions in Table 4 (Example 2 and Comparative examples 8-10) were prepared using conventional manufacturing processes and actual application test using each of Example 2 and Comparative examples 8-10 were carried out on human for two months. For each experiment, a group of twenty women with notably dry skin, wrinkles and the like from their late thirties to fifties were respectively selected. These compositions were applied to their face two times a day (once in the morning and once in the evening) for two months, and their skin conditions before and after theses experiments were evaluated according to the criteria as follows: "improvement", "slight improvement" and "no change". The results of the experiments are shown in Table 5 as the total of subjects in each evaluation.

**Table 4**

| | Example 2 | Comparative example 8 | Comparative example 9 | Comparative example 10 |
|---|---|---|---|---|
| retinol palmitate | 0.1 | 0.1 | - | 0.1 |
| hinokitiol | 0.001 | 0.001 | 0.001 | - |
| phytosterol | 1.0 | - | 1.0 | 1.0 |
| ceramide III | 0.01 | - | 0.01 | 0.01 |
| meadow-foam oil | 0.1 | - | 0.1 | 0.1 |
| hydroxyethyl-cellulose | 0.8 | 0.8 | 0.8 | 0.8 |
| glycerin | 15.0 | 15.0 | 15.0 | 15.0 |
| paraben | 0.2 | 0.2 | 0.2 | 0.2 |
| polyglycerin fatty acid ester | 3.0 | 3.0 | 3.0 | 3.0 |
| ester oil | 8.0 | 8.0 | 8.0 | 8.0 |
| squalane | 2.0 | 2.0 | 2.0 | 2.0 |
| plant extract | 0.5 | 0.5 | 0.5 | 0.5 |
| flavor | 0.03 | 0.03 | 0.03 | 0.03 |
| purified water | balance | balance | balance | balance |

| | | | | |
|---|---|---|---|---|
| (The units in the table are weight %.) | | | | |

**Table 5**

| | | Example 2 | Comparative example 8 | Comparative example 9 | Comparative example 10 |
|---|---|---|---|---|---|
| dry skin | improvement | 5 | 0 | 0 | 0 |
| | Slight improvement | 15 | 4 | 6 | 8 |
| | no change | 0 | 16 | 14 | 12 |
| skin tautness and elasticity | improvement | 4 | 0 | 0 | 0 |
| | slight improvement | 16 | 3 | 2 | 7 |
| | no change | 0 | 17 | 18 | 13 |
| wrinkles | improvement | 2 | 0 | 0 | 0 |
| | slight improvement | 18 | 3 | 1 | 5 |
| | no change | 0 | 17 | 19 | 15 |

The results of Table 5 revealed that Example 2 according to the present invention was much more effective at improving dry skin, skin tautness and elasticity loss, wrinkles, and the like, compared with Comparative examples 8-10. Also, no skin epispastic reaction and skin sensitizing reaction against the compositions of the above Examples 1 and 2 occurred in the subjects.

The prescription examples of the external dermal agents including the vitamin A compound, the hinokitiol and the epidermal lipid according to the present invention are shown as follows.

| Example 3 Skin lotion | |
|---|---|
| retinol palmitate | 0.01 (wt %) |
| hinokitiol | 0.001 |
| hydrogenated soybean phospholipid | 0.1 |
| hydroxyethylcellulose | 0.1 |
| 1,3-butyleneglycol | 3.0 |
| paraben | 0.2 |
| polyoxyethylene hydrogenated castor oil | 0.3 |
| alcohol | 10.0 |
| plant extract | 0.5. |
| flavor | 0.03 |
| purified water | balance |

| Example 4 Milky lotion | |
|---|---|
| retinol acetate | 0.1 (wt %) |
| hinokitiol . | 0.01 |
| phosphatidylethanolamine | 0.5 |
| ceramide 3 | 0.01 |
| carboxyvinylpolymer | 0.3 |
| 1,3-butyleneglycol | 5.0 |
| paraben | 0.2 |
| polyoxyethylene hydrogenated castor oil | 0.1 |
| polyglycerin fatty acid ester polyoxyethylene sorbitan fatty acid ester 0.3 | 1.0 |
| liquid paraffin | 3.0 |
| potassium hydroxide | 0.07 |
| plant extract | 0.5 |
| flavor | 0.03 |
| purified water | balance |

| Example 5 Cream | |
|---|---|
| retinol | 0.1 (wt %) |
| hinokitiol | 0.1 |
| cholesterol | 1.0 |
| lysophosphatidyl choline | 0.1 |
| olive oil | 2.0 |
| glycerine | 15.0 |
| paraben | 0.2 |
| carboxyvinylpolymer | 0.3 |
| polyoxyethylene hydrogenated castor oil | 0.1 |
| monoglycerin fatty acid ester | 5.0 |
| propylene glycol fatty acid ester | 5.0 |
| polyethylene glycol fatty acid ester | 0.5 |
| squalane | 10.0 |
| behenyl alcohol | 3.0 |
| stearic acid | 1.0 |
| plant extract | 0.5 |
| flavor | 0.03 |
| purified water | balance |

| Example 6 Essence | |
|---|---|
| retinol acetate | 0.1 (wt %) |
| hinokitiol | 0.001 |
| safflower oil | 1.0 |
| ceramide 3 | 0.01 |
| hydroxyethylcellulose | 0.6 |
| dipropyleneglycol | 10.0 |
| paraben | 0.2 |
| polyoxyethylene hydrogenated castor oil | 0.1 |
| polyglycerin fatty acid ester | 2.0 |
| polyoxyethylene sorbitan fatty acid ester 0.5 | |
| squalane | 5.0 |
| stearic acid | 1.0 |
| plant extract | 0.5 |
| flavor | 0.03 |
| purified water | balance |

As described above, the external dermal agents according to the present invention provide superior preventions and improvements to dry skin, rough skin, wrinkles and the like, loss of skin tautness and elasticity compared with the other conventional compositions.

## Claims

1. An external dermal agent for skin treatment comprising :
(A) 0,001 - 5,0 wt % of a vitamin A compound and/or a derivative thereof;
(B) 0,0001 - 2,0 wt % of a hinokitiol and/or a derivative thereof ; and
(C) 0,01 - 20,0 wt % of an epidermal lipid,
wherein the vitamin A compound and/or the derivative thereof is a compound having vitamin A activity, the hinokitiol and/or the derivative thereof is a tropolone compound or a derivative thereof (beta-thujaplicin), and the epidermal lipid is at least one selected from common lipids existing in skin.

## Patentansprüche

1. Äußerliches dermales Mittel zur Hautbehandlung umfassend:
(A) 0,001 - 5,0 Gew.-% einer Vitamin A Verbindung und/oder eines Derivats davon;
(B) 0,0001 - 2,0 Gew.-% eines Hinokitiols und/oder eines Derivats davon; und
(C) 0,01 - 20,0 Gew.-% eines epidermalen Lipids,
wobei die Vitamin A Verbindung und/oder das Derivat davon eine Verbindung mit Vitamin A Aktivität ist, das Hinokitiol und/oder das Derivat davon eine Tropolonverbindung oder ein Derivat davon (beta-Thujaplicin) ist, und das epidermale Lipid mindestens eines ist, das aus herkömmlichen Lipiden, die in Haut vorkommen, ausgewählt ist.

## Revendications

1. Agent dermique externe destiné au traitement de la peau, comprenant :
(A) 0,001 à 5,0 % en poids d'un composé de vitamine A et/ou d'un dérivé de celui-ci ;
(B) 0,0001 à 2,0 % en poids d'un hinokitiol et/ou d'un dérivé de celui-ci ; et
(C) 0,01 à 20,0 % en poids d'un lipide épidermique,
dans lequel le composé de vitamine A et/ou le dérivé de celui-ci est un composé ayant une activité de vitamine A, l'hinokitiol et/ou le dérivé de celui-ci est un composé de tropolone ou un dérivé de celui-ci (béta-thujaplicine) et le lipide épidermique est au moins un lipide choisi parmi les lipides communs existant dans la peau.
